Europäisches Patentamt

European Patent Office

Office européen des brevets

(11) Publication number: **0 387 065**
**A2**

## EUROPEAN PATENT APPLICATION

(21) Application number: 90302492.5

(51) Int. Cl.5: **C12N 5/00, C12M 3/00**

(22) Date of filing: 08.03.90

(30) Priority: 08.03.89 JP 53716/89
09.05.89 JP 116251/89
12.09.89 JP 234636/89
12.09.89 JP 234637/89

(43) Date of publication of application:
**12.09.90 Bulletin 90/37**

(84) Designated Contracting States:
**AT BE CH DE DK ES FR GB GR IT LI LU NL SE**

(71) Applicant: **MITSUI PETROCHEMICAL
INDUSTRIES, LTD.**
**2-5, Kasumigaseki 3-chome Chiyoda-ku
Tokyo 100(JP)**

(72) Inventor: **Sakai, Masao, c/o Mitsui
Petrochemical
Industries Ltd., 1-2 Waki 6-chome, Waki-cho
Kuga-gun, Yamaguchi-ken(JP)**
Inventor: **Yukimune, Yukimune, c/o Mitsui
Petrochemical
Industries Ltd., 1-2 Waki 6-chome, Waki-cho
Kuga-gun, Yamaguchi-ken(JP)**
Inventor: **Deno, Hiroshi, c/o Mitsui
Petrochemical
Industries Ltd., 1-2 Waki 6-chome, Waki-cho
Kuga-gun, Yamaguchi-ken(JP)**

(74) Representative: **Myerscough, Philip Boyd et al
J.A.Kemp & Co. 14, South Square Gray's Inn
London, WC1R 5EU(GB)**

(54) Method for culturing plant tissue, apparatus therefor and method for producing metabolite.

(57) A method for culturing a tissue of a plant in a culture tank to which a liquid medium having a dissolved oxygen concentration of 10ppm or more in a flow state of giving substantially no vibration to the tissue or the cell and an apparatus for culturing a tissue of a plant according to the foregoing method. In this apparatus, a means for supplying oxygen or a gas containing oxygen to a liquid medium in an aeration tank and a means for forcedly supplying the obtained oxygen-dissolved liquid medium to a culture tank are provided, where the oxygen-dissolved liquid medium is supplied to the culture tank in a flow state of giving substantially no vibration to the tissue or the cell in the culture tank, particularly preferably in the form of a uniform piston flow. This invention is applied to the mass proliferation of a cell and the production of a metabolite such as alkaloid or the like because a tissue or a cell of a plant such as an adventitious root, a callus, etc. are cultured efficiently at a high proliferation speed and at a high density by these method and apparatus.

# METHOD FOR CULTURING PLANT TISSUE, APPARATUS THEREFOR AND METHOD FOR PRODUCING METABOLITE

## BACKGROUND OF THE INVENTION

This invention relates to a method for culturing a plant tissue, a method for producing a metabolite by employing the foregoing method for culturing a plant tissue and an apparatus for culturing a plant tissue. In further detail, this invention relates to a method for high density culture of a plant tissue and an apparatus for culturing a plant tissue suitable for the production of a metabolite.

As alkaloids which are one of metablites, for example, scopolamine is held to be important as an anntispasmodic, an analgesic, etc., hyoscyamine is held to be important as a parasympatholytic agent and berbeline is held to be important as an agent for stomatic and intestinal disorders. These compounds are produced by the extraction from natural plants. However, the compounds have problems such as the production thereof is influenced by the weather, the harvesting time thereof is restricted, etc. because natural products are used as raw materials. Owing to such circumstances, many studies are made of the production of these compounds by plant tissue culture [e.g., *Plant Cell Reports*, vol. 1, pp. 101~103 (1982)].

The present inventors found that the productivity of alkaloids was improved by increasing the dissolved oxygen concentration in the plant tissue culture by supplying oxygen to a culture tank [Japanese Patent Application Kokai (Laid-open) Nos. 6674/1987 and 6676/1987]. However, they found that satisfactory results could not be obtained by this method because, when the density of tissues to be cultured was increased in order to further improve the productivity of alkalids, bubbles stayed in gaps between the tissues to be cultured to hinder the smooth supply of oxygen and nutrients.

On the other hand, a method for supplying an air-supplied medium to a culture tank has been proposed [Japanese Patent Application Kokai (Laid-open) No. 179383/1987]. However, this method has a disadvantage in that the consentration of oxygen to be supplied to tissues to be cultured is insufficient in respect of the high density plant tissue culture or the productivity of metabolites.

As described above, the conventional plant tissue culture methods have various problems particularly in respect of the high density tissue culture on an industrial scale. In addition, these methods could not necessarily give a satisfactory yield of metabolites in case of industrially producing the metabolites by tissue culture.

Therefore, it has been important problems to develop a plant tissue culture method by which not only a plant can be cultured at a high density on an industrial scale but also the productivity of metabolites can be improved and an apparatus for operating this method.

## SUMMARY OF THE INVENTION

The present inventors made intensive studies in order to overcome the disadvantages of the conventinal methods. As the result, they found that, tissues of a plant could be cultured at a density higher than the conventional methods in case of culturing tissues or cells of a plant in a culture tank to which a liquid medium having a dissolved oxygen concentration of 10ppm or more, that tissues of a plant could be cultured at an unexpected high density in case of carrying out the aforementioned plant tissue culture while supplying a liquid medium in a flow state of giving substantially no vibration to tissues or cells of a plant, and that the productivity of metabolites could be strikingly improved in case of applying this plant tissue culture method to the production of metabolites.

Furthermore, the present inventors found that the above problems could be solved advantageously by, as a means of the plant tissue culture, adopting an apparatus for plant tissue culture so constructed as to obtain an oxygen-dissolved liquid medium by supplying oxygen or a gas containing oxygen to an aeration tank for a liquid medium and then forcedly supply the obtained oxygen-dissolved liquid medium to a tank for culturing tissues or cells of a plant and then culturing the tissues or the cells of a plant by using this plant tissue culture apparatus under a predetermined culture conditions.

On the basis of the abovementioned novel findings, the present inventors intensively made a wider and deeper study thereof to complete the present invention.

That is, the present invention purposes to provide a plant tissue culture method featured by culturing tissues or cells of a plant in a culture tank to which a liquid medium having a dissolved oxygen concentration of 10ppm or more is supplied.

The present invention further purposes to provide a plant tissue culture apparatus featured by having a means of supplying oxygen or a gas containing oxygen to an aeration tank for obtaining an oxygen-dissolved liquid medium and a means of forcedly supplying the oxygen-dissolved liquid medium into a culture tank for culturing tissues or cells of a plant from the aforementioned aeration tank.

The present invention still further purposes to provide a method for producing a metabolite featured by culturing tissues or cells of a plant and then collecting a metabolite from the obtained culture.

In the present invention, a gas containing oxygen is not supplied to a medium directly. Therefore, oxygen can be supplied sufficiently without giving no damage to cells of a plant producing the aimed metabolite, so that not only the proliferation speed and the high density culture of cells can be attained but also the production of metabolites such as alkaloid and the like is accelerated to enable the efficient production thereof. In addition, the present invention enables the high density proliferation of tissues or cells of a plant such as an adventitious root, a callus, etc. particularly by culturing the same while supplying the predetermined highly concentrated oxygen-dissolved liquid medium in a state of giving no vibration to the aforementioned tissues or cells. Therefore, the present invention can be applied to the mass propagation of a plant very effectively. In addition, in case of applying to the production of metabolites such as alkaloids, the present invention can improve the productivity thereof by leaps and bounds.

## BRIEF DESCRIPTION OF THE DRAWINGS

Fig. 1 shows one embodiment of the present plant tissue culture apparatus.

Fig. 2 shows another embodiment of the present plant tissue culture apparatus.

Fig. 3 shows a comparative example of a plant tissue culture apparatus having no means of forcedly supplying an oxygen-dissolved liquid medium into a culture tank from an aeration tank.

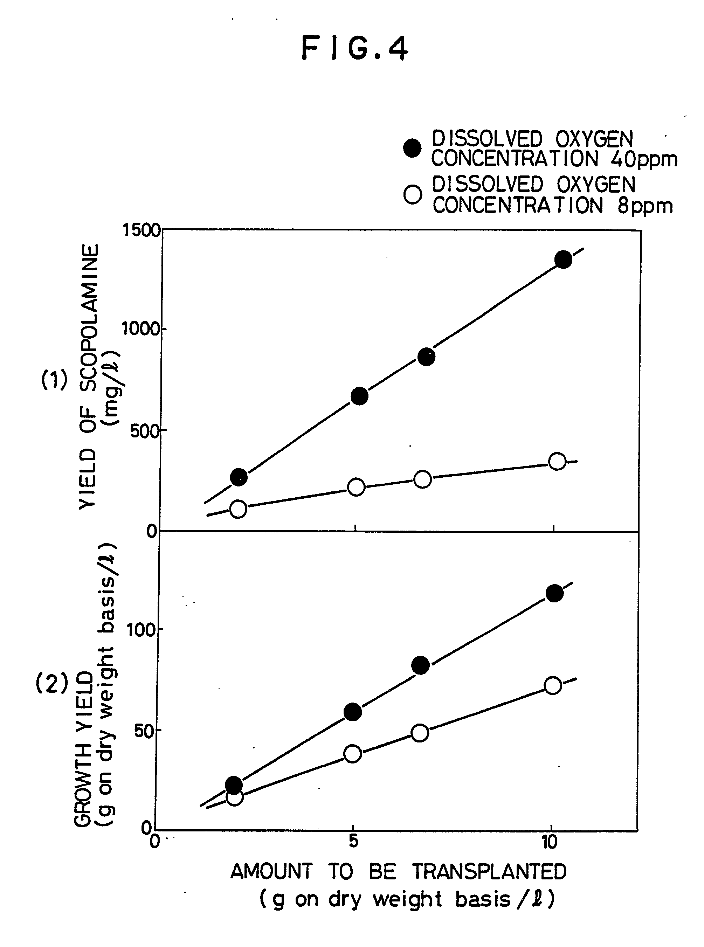

(1) and (2) of Fig. 4 are graphs respectively showing the relationship between the amount to be transplanted and the yield of scopolamine and the relationship between the amount to be transplanted and the growth yield in an example of the present invention and a comparative example.

## DETAILED DESCRIPTION OF THE PRESENT INVENTION

As previously described, the present invention first contemplates providing a method for culturing a tissue or a cell of a plant in a culture tank to which a liquid medium having a dissolved oxygen concentration of 10ppm or more.

It is preferred that the aforementioned dissolved oxygen concentration is preferably 15ppm or more and that the aforementioned culture is carried out by continuously or intemittently supplying a liquid medium to tissues or cells of a plant in a culture tank in a flow state of giving substantially no vibration to the tissues or the cells. Whereby, the tissues or the cells of a plant can be not only cultured without giving no damage thereto as same as in case of using the conventional agitation-type culture tank and airlift-type culture tank but also at a high density, for example, of 400g on fresh weight basis/$\ell$ or more.

As preveously described, the present invention further contemplates providing a plant tissue culture apparatus having a means of supplying oxygen or a gas containing oxygent to an aeration tank for obtaining an oxygen-dissolved liquid medium and a means of forcedly supplying the oxygen-dissolved liquid medium into a culture tank for tissues or cells of a plant from the aforementioned aeration tank.

In the present tissue culture apparatus, it is preferred that at least a means of preventing the flow of tissues or cells of a plant to be cultured, for example, a filter, at the side of exhausting the oxygen-dissolved liuqid medium in the culture tank.

In addition, the means of forcedly supplying the oxygen-dissolved liquid medium into the culture tank from the outside is preferably a means of supplying the liquid medium to tissues or cells to be cultured in a flow state of giving substantially no bibration thereto, particularly preferably a means of supplying the liquid medium to tissues or cells of a plant by uniform piston flow.

Furthermore, in case that tissues or cells of a plant are those having photosynthetic ability and the culture thereof is carried out under lightening, it is preferred to use a culture tank made of a transparent material.

As the aforementioned flow state of giving substantially no vibration to tissues or cells, a uniform piston flow is particularly preferable in the present method and apparatus.

Furthermore, it is considerably desirable for carrying out the plant tissue culture at a high density that a liquid medium is supplied by the forced supply means.

As preveously described, the present invention still further contemplates a method for producing a metabolite by carrying out the culture according to the above plant tissue culture method and then harvesting the aimed metabolite from the obtained cultures.

As plants usable in the present ivention, any plants having cells producing a metabolite will do. It is no matter that the cells are differentiated into organs such as an adventitious root and the like. In addition, a callus or a suspended cell can be used similarly.

As specific examples of a plant to which the present invention is applied, plants belonging to the family *Solanaceae* such as plants belonging to the genus *Duboisia*, e.g., *Duboisia myoporoides, Duboisia leichhardtii*, etc.; plants belonging to the genus *Datura*, e.g., *Datura tatula, Datura arborea, Datura stramonium*, etc.; plants belonging to the genus *Scopolia*, e.g., *Scopolia japonica*, etc.; plants belonging to the genus *Hyoscyamus*, e.g., *Hyoscyamus niger*, etc. and plants belonging to the genus *Atropa*, e.g., *Atropa belladonna*, etc.; plants belonging to the genus *Coptis*, e.g., *Coptis japonica* Makino, *Coptis japonica* Makino var. *dissecta* Nakai, *Coptis japonica* Makino var. *japonica, Coptis japonica* Makino var. *major* Satake, *Coptis quinquefolia* Miq., *Coptis trifolia* Salisb., etc.; plants belonging to the genus *Thalictrum*, e.g., *Thalictrum minus* L. var. *hypoleucum* Miq., etc.; plants belonging to the genus *xanthoriza*; plants belonging to the genus *Hydrastis*; etc. can be enumerated.

In the present plant tissue culture method and the present method for producing a metabolite, it is required that tissues or cells of the aforementioned plant are cultured in a culture tank to which a liquid medium having a dissolved oxygen concentration of 10ppm or more is supplied. In case that the dissolved oxygen concentration is less than 10ppm, the dissolved oxygen concentration in the liquid medium cannot be increased to a concentration necessary for the high density tissue culture and for increasing the yield of a metabolite. Furtheremore, it is preferred that the aforementioned dissolved oxygen concentration is 15ppm or more.

As preferable example of a gas containing oxygen which can be used in the present invention, a gas prepared by adding pure oxygen to air or pure oxygen itself can be enumerated.

As the culture tank to which a liquid medium having a dissolved oxygen concentration of 10ppm or more in the aforementioned method, any culture tank can be used without restriction so far as the aforementioned gas is not directly supplied inside and the previously aerated liquid medium can be supplied to. Thus, an oxygen-dissolved liquid medium can be forcedly supplied inside a culture tank.

Hereinafter, the present invention will be described, referring to the attached drawings.

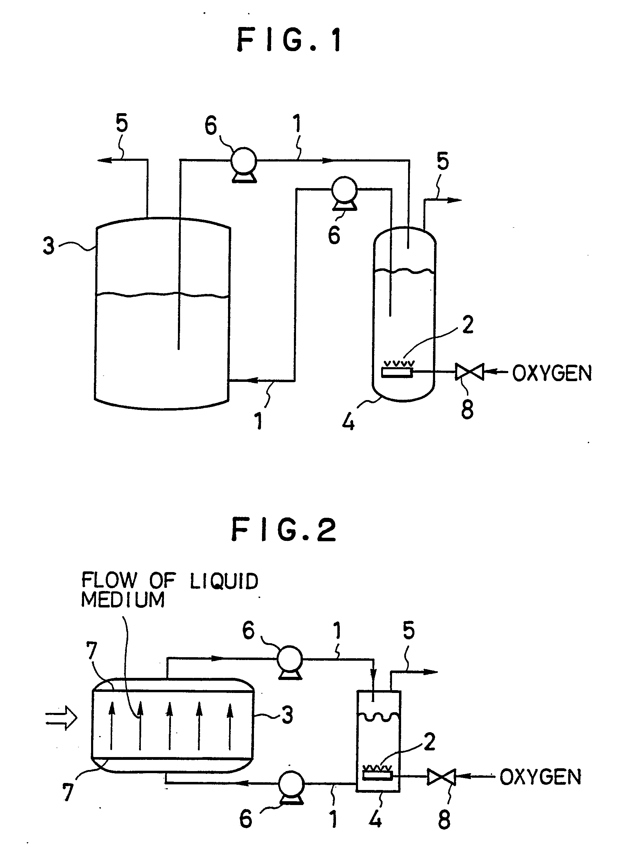

In Fig. 1, an aeration tank 1 and a culture tank 3 are separately provided and connected with a pipe 1 for supplying. In the aeration tank 4, a gas containing oxygen is supplied to a liquid medium through a tube 2 for supplying the gas containing oxygen. A liquid medium made to have a dissolved oxygen concentration of 15ppm or more is forcedly supplied to the culture tank 3 by pumps 6,6 through the pipe 1 for supplying a liquid culture.

Incidentally, the numeral 5 indicates an exhaustion pipe for exhausting oxygen gas after aeration and the numeral 8 indicates a valve.

In the embodiment of Fig. 1, a culture tank and an aeration tank are connected by installing a pipe therebetween. However, these tanks may be adjacently arranged by using a filter, a mesh, etc. as a partition. As a method for supplying the aforementioned gas containing oxygen, a fileter, a sintered metal or the like can be used.

In the present invention, the supply of the aforemen tioned liquid medium in a flow state of giving substantially no vibration to tissues or cells means that the liquid medium is generally supplied forcedly inside a culture tank from one direction in an almost steady flow state by a forced supply means such as a pump 6 shown in Fig. 1 without accompanying the conventional supply of a liquid medium into a culture tank from small holes by blowing or operations giving a vibration to tissues or cells such as stirring and the like of the aforementioned liquid medium in a culture tank and that the vibration of tissues or cells of an adventitious root and the like is hardly observed visually when the liquid medium is supplied. In this case, the liquid medium is supplied into a culture tank at a speed in the range of 1m$\ell$/$\ell$/min. to 50$\ell$/$\ell$/min., preferably in the range of 500m$\ell$/$\ell$/min. to 10$\ell$/$\ell$/min.

The aforementioned liquid medium having a dissolved oxygen concentration of 10ppm, preferably of 15ppm is supplied to tissues or cells of an advantitious root, a callus or the like in the aforementioned culture tank in such a flow state as described above. Whereby, the tissues or the cells in the culture tank become close each other and then come into almost one solid body on the whole. Therefore, it becomes possible to proliferate a tissue or a cell at a high density such as 400g on the fresh weight basis/$\ell$ or more or 20g on the dry weight basis/$\ell$ or more, as was unat tainable by the conventional techniques. In addition, it becomes possible to increase the productivity of a metabolite by leaps and bounds.

Furthermore, in case of carrying out the aforementioned culture of tissues or cells in a space having a capacity limited by, e.g., a filter or the like fixed in a culture tank, the increase in the number of cells by cell

4

division proceeds with the continuation of the culture. However, the increase in the volume of the whole tissue cultures is suppressed by the aforementioned limited capacity of a space, so that a dry weight/fresh weight ratio and a dry weight of individual cells become extraordinarily high. Therefore, the high density tissue culture exceeding the conventional technical commonsense can be attained and, in its turn, the productivity of a metabolite can be improved by leaps and bounds. It has been confirmed that, in case of applying the aforementioned method for culturing tissues or cells in a space having a limited capacity to, e.g., an adventitious root of *Duboisia myoporoides*, cultures having a dry weight/fresh weight of 0.07 or more and a density as high as 40g on dry weight basis/$\ell$ or more can be obtained.

An apparatus shown in Fig. 2 is constructed similarly to the culture appartus of Fig. 1, except that a pump 6 for proliferating tissues or cells at a high density in a culture tank 3 and for forcedly supplying a liquid medium to the tissues or the cells occupying the inside of the culture tank and stainless meshes 7,7 fixed to the inside wall of the culture tank 3 at predetermined distances in order to not only preventing the vibration of the tissues or the cells at time of supplying the liquid medium but also culturing the aforementioned tissues or cells in a space having a limited capacity are provided. The apparatus shown in Fig. 2 can be advantageously used for the tissue culture of an adventitious root and the production of its metablite thereby.

A liquid medium to be used in the present invention is the one which contains inorganic ingredients and carbon sources as essential ingredients and is adjusted by adding plant hormones and vitamins and, at need, amino acids.

As the inorganic ingredients, inorganic salts containing elements such as nitrogen, phosphorous, potassium, sodium, calcium, magnesium, sulfur, iron, manganese, zinc, boron, molybdenum, chlorine, iodine, cobalt, etc. can be enumerated. As specific examples thereof, compounds such as potasium nitrate, sodium nitrate, ammonium nitrate, ammonium chloride, potassium chloride, calcium chloride, potassium hydrogenphosphate, potassium dihydrogenphosphate, magnesium sulfate, magnesium chloride, sodium sulfate, ferrous sulfate, ferric sulfate, manganese sulfate copper sulfate, sodium molybdate, molybdenum trioxide, potassim iodide, zinc sulfate, boric acid, cobalt chloride, etc. can be enumerated.

As examples of carbon sources of the aforementioned liquid medium, carbohydrates such as sucrose and the like or their derivatives, organic acids such as fatty acid and the like, primary alcohols such as ethanol and the like, etc. can be enumerated.

As examples of plant hormones of the aforementioned liquid medium, auxins such as 1-naphthaleneacetic acid (NAA), 3-indole-acetic acid (IAA), *p*-chlorophenoxy acetic acid, 2,4-dichlorophenoxyacetic acid (2,4-D), indole-3-butyric acid (IBA), their derivatives, etc. and cytokinins such as benzyladenine (BA), kinetin, zeatin, etc. can be enumerated.

As examples of vitamins of the aforementioned liquid medium, biotin, thiamine (vitamin $B_1$), pyridoxin (vitamin $B_6$), pyridoxal, pyridoxamine, calcium pantothenate, ascorbic acid (vitamin C), inositol, nicotinic acid, nicotinamide, riboflavin (vitamin $B_2$), etc. can be enumerated.

As examples of amino acids of the aforementioned liquid medium, glycine, alanine, glutamic acid, cystine, phenylalanine, lysine, etc. can be enumerated.

It is desirable that the aforementioned liquid medium of the present invention is used generally by adding the aformentioned ingredients to the following concentrations:

| Inorganic ingredients | approx. 0.1$\mu$M ~ approx. 100mM |
|---|---|
| Carbon sources | approx. 1g/$\ell$ ~ approx. 100g/$\ell$ |
| Plant Hormones | approx. 0.01$\mu$M ~ approx. 100$\mu$M |
| Vitamins | approx. 0.1mg/$\ell$ ~ approx. 150mg/$\ell$ |
| Amino acids | approx. 0 ~ approx. 100mg/$\ell$ |

As specific examples of the aforementioned liquid medium to be used for the present plant tissue culture, media to be prepared by adding the aforementioned carbon sources, plant hormones and, at need, the aforementioned vitamins and amino acids to media such as Murashige-Skoog medium, Linsmaier-Skoog (RM-1965) medium, White's ('63) medium, Gamborg's B-5 medium, Mitsui's M-9 medium, Nitsch-Nitsch medium, etc. can be enumerated, among which the one to be prepared by using Nitsch-Nitsch medium, Linsmaier-Skoog medium or Murashige-Skoog medium is particularly preferable in the present invention. Incidentally, the compositions of the above conventionally known media are described, for example, in *"New Plant Tissue Culture"* [Takeuchi, Nakajima and Furuya, pp. 386~391, Asakura Shoten (1979)].

In the present invention, the plant tissue culture is carried out by using the aforementioned liquid medium in the aforementioned culture tank to thereby obtain cultured cells or cultured tissued containing the aimed metabolite.

As specific examples of tissues of the aforementioned plants to be used in the present tissue culture, cultured cells or cultured tissues of the plant to be obtained by the present tissue culture methods or by other conventional tissue culture methods can be enumerated in addition to roots, leaves, stems, seeds, flower buds, etc. In case of using a plant belonging the the genus *Duboisia*, it is particularly preferred that an adventitous root to be obtained by previously culturing tissues of the plant is cultured in the aforementioned culture tank. In this case, a material adventitious root is cultured to proliferate according to the present method to thereby give adventitious roots containing a large amount of tropane alkaloids such as scopolamine, hyoscyamine, etc. as metabolites.

As examples of a metabolite to be produced by the present invention, tropane alkaloids (scopolamine, hyoscyamine, etc.), isoquinoline alkaloids (berberine, etc.), etc. can be enumerated.

In order to isolate a metabolite from cultured cells or cultured tissues containing the metabolite in the present invention, ordinary methods for isolating and purifying a metabolite described, for example, in the pharmacopoeia and the like can be employed.

EXAMPLES

Hereinafter, the present method will be described more specifically, referring to the examples.

Example 1

Leaves of *Duboisia myoporoides* R.Br cultivated in a harbary of our company were washed, immersed in 10% antiformin solution for 10 minutes. After washing 3 times with sterile water, the leaves were cut into approx. 1-cm pieces, placed on a Linsmaier-Skoog agar medium to which 1-naphthaleneacetic acid and benzyladenine were respectively added to concentrations of $10^{-5}$ M and $10^{-6}$ M and then cultured at 25°C for 30 days. Adventitious roots generated simultaneously with the formation of calluses were cut out, transplanted to a Nitsch-Nitsch medium to which sucrose and indole-3-butyric acid were added respectively to concentrations of 3% and $10^{-5}$ M and then subcultured for 2 years.

100mg (dry weight) of thus obtained advantitious root was transplanted into a culture tank of a culture apparatus consisting of an aeration tank and the culture tank shown in Fig. 1 and then cultured for 3 weeks according to a culture method by which a liquid medium (containing 3% sucrose and $10^{-5}$ M indole-3-butyric acid, dissolved oxygen concentration = 40ppm) supplied with pure oxygen in the aeration tank was supplied to a culture tank containing 50mℓ of Nitsch-Nitsch medium at a flow rate of 20mℓ/min. The obtained adventitious roots were dried and then extracted with 50mℓ of basic chloroform-methanol solution. Subsequently, 40mℓ of 1N sulfuric acid was added thereto to move the alkaloid layer to sulfuric acid layer. Furthermore, 2mℓ of ammonia water and 40mℓ of chloroform were added thereto to move the alkaloid to chloroform layer, followed by concnetration under reduced pressure. The concentrated layer was gas chroatographed to analyze the amount of alkaloid. The yield of scopolamine in this case was shown in Table 1. Incidentally, the gas chromatographic analysis was carried out under the following conditions:
Column: Silicone OV-17 (1%) on Chromosorb W (mesh 80~100), 3mm∅ × 1m glass column
Carrier gas: N₂
Column temperature: 200°C

Example 2

Adventitious roots of *Duboisia myoporoides* R. Br were cultured in the same manner as in Example 1, except that a gas to be supplied was prepared by adding oxygen to air and the gas had an oxygen partial pressure of 50%. Then, scopolamine was extracted from the culture and its content was measured. The results were given in Table 1.

Example 3

The culture was carried out in the same manner as in Example 1, except that a gas having an oxygen partial pressure of 30% was supplied instead of pure oxygen. The re sults were given in Table 1.

Comparative Example 1

The culture was carried out in the same manner as in Example 1, except that air was supplied instead of pure oxygen. The results were given in Table 1.

Comparative Example 2

The culture was carried out in the same manner as in Example 1, except that pure oxygen was directly supplied to the culture tank. The results were given in Table 1.

Table 1

| | Cell | Gas | Dissolved Oxygen Concentration (ppm) | Growth Yield (g**/ℓ) | Scopolamine Content (wt %) |
|---|---|---|---|---|---|
| Example 1 | Adventitious root* | Pure oxygen | 40 | 20 | 1.3 |
| Example 2 | " | 50% Partial oxygen pressure | 20 | 20 | 1.2 |
| Example 3 | " | 30% Partial oxygen pressure | 12 | 16 | 0.8 |
| Comparative Example 1 | " | Air | 8 | 16 | 0.7 |
| Comparative Example 2 | " | Pure oxygen, directly to culture tank | 40 | 16 | 0.8 |

* Adventitious roots of *Duboisia myoporoides*.

** On dry weight basis.

8

Comparative Example 3

The adventitious roots of *Duboisia myoporoides* used in Example 1 were those obtained by a 2-year subculture. In this comparative example, however, those obtained by a 6-month subculture under the same condition in Example 1 were used. That is, this comparative example is a test carried out 1.5 years before the Example 1, and thus, the adventitious roots of Example 1 and Comparative Example 3 are those of the same cell line having the same parent cell.

In this comparative example, 0.8g (dry weight) of adventitious roots obtained by the abovementioned 6-month subculture were transplanted to an aeration-culture tank provided with a dissolved oxygen concentration regulator and a glass filter at the bottom for aeration and containing 1 ℓ of the above liquid medium, followed by culturing for 3 weeks while keeping the dissolved oxygen concentration of the liquid medium at values given in Table 2. The obtained adventitious roots were dried and then extracted with 1 ℓ of chroloform-methanol solution, followed by addition of 1 ℓ of 1N sulfuric acid to move alkaloid to the sulfuric layer. Furthermore, 100m ℓ of ammonia water and 1 ℓ of chloroform were added thereto to move the alkaloid to the chloroform layer, followed by concentration under reduced pressure. The extract was analyzed by gas chromatography to obtain the total amount of tropane alkaloids such as scopolamine, hyos cyamine, acetylropine, isobutyroyltropine, valeroyltropine and tigloidine per dry weight and the content of scopolamine per dry weight were obtained. The results were given in Table 2.

Table 2

| Test No. | Dissolved Oxygen Concentration (ppm) | Total Tropane Alkaloid (%)* | Scopolamine Content (%)* |
|---|---|---|---|
| 1 | 20 | 2.49 | 0.71 |
| 2 | 30 | 2.95 | 0.82 |
| 3 | 40 | 3.05 | 0.85 |
| 4 | 50 | 2.63 | 0.75 |
| 5 | 60 | 2.13 | 0.61 |
| 6 | 4 | 1.86 | 0.49 |
| 7 | 8 | 2.24 | 0.57 |

* % by weight per dry weight.

From the comparison of the results of the above example and comparative example, it is obvious that both the content and the production of scopolamine are low even by increasing the dissolved oxygen concentration in case of the known stirring culture, whereas high content and production of scopolamine can be obtained only when the present culture method giving no vibration to tissues or cells is adopted.

Example 4

Cultured cells of *Coptis japonica* were cultured in the same manner as in Example 1, except that a medium to be used was a Linsmaier-Skoog liquid medium containing 100μM 1-naphthaleneacetic acid and 5μM benzyladenine as plant hormones. Then, the berberine was extracted from the culture with 90% methanol, the content of which was measured by using HPLC. The results were shown in Table 3.

Comparative Example 4

The culture of cultured cells of *Coptis japonica* and the extraction and the content measurement of berberine were carried out in the same manner as in Example 4, except that air was used as a gas to be supplied. The results were given in Table 3.

## EP 0 387 065 A2

Table 3

| | Cell | Gas | Dissolved Oxygen Concentration (ppm) | Growth Yield (g**/ℓ) | Berberine Content (wt %) |
|---|---|---|---|---|---|
| Example 4 | Cultured cells* | Pure oxygen | 40 | 30 | 13.5 |
| Comparative Example 4 | " | Air | 8 | 24 | 8.5 |

\* Cultured cells of *Coptis japonica*.
\*\* On dry weight basis.

Comparative Example 5

Leaves of *Coptis* var. *dissecta* Nakai were sterilized with 70% ethanol solution and an aqueous solution of sodium hypochlorite (available chlorine concentration, 0.5%), placed on solid agar medium (agar content, 1 wt %) prepared by solidifying a sterile Linsmaier-Skoog liquid medium containing 3% sucrose, $10^{-5}$ M 1-naphthaleneacetic acid and $10^{-8}$ M benzyladenine and then cultured at 25°C in a dark place to obtain calluses of *Coptis japonica* var. *dissecta* Nakai. Then, the calluses were transplanted to fresh Linsmaier-Skoog media at 14-day intervals under the same conditions as above and gyratorily cultured in a rotary shaker (amplitude, 25mm; 100 rpm) to increase the growth rate. Whereby, the stabilized cells of *Coptis japonica* Makino var. *dissecta* Nakai were obtained.

1.5ℓ of sterilized above liquid medium in which $Cu^{2+}$ concentration was changed to 1μM and 15g on fresh weight basis of above calluses were placed in an aeration-stirring tank (capacity, 2ℓ) provided with a dissolved oxygen densitometer and a pipe for supplying a gas containing oxygen. The calluses were culture for 2 weeks at 25°C in a dark place while adjusting the amount of gas containing oxygen to be supplied to values given in Table 4. After drying the obtained calluses, the dried calluses were crushed in a porcelain mortar and then extracted with 90% ethanol to obtain isoquinoline alkaloids. This extract was subjected to high performance chromatography to analyze into isoquinoline alkaloids such as berberine, palmatine, coptisine, jateorrhizine. The results were given in Table 4.

Table 4

| Test No. | Dissolved Oxygen Concentration (ppm) | Yield of Callus (g*/ℓ) | Total Isoquinoline alkaloid (wt %) | Content of Berberine (wt %) |
|---|---|---|---|---|
| 1 | 4 | 11.0 | 7.4 | 6.7 |
| 2 | 8 | 11.5 | 10.7 | 7.2 |
| 3 | 10 | 11.9 | 12.7 | 8.1 |
| 4 | 15 | 12.3 | 12.5 | 8.0 |
| 5 | 20 | 11.8 | 11.3 | 7.6 |
| 6 | 30 | 6.5 | 6.3 | 4.4 |
| 7 | 40 | 2.3 | 3.9 | 2.6 |

\* On dry weight basis.

The results of this test also suggests that preferable results cannot be obtained by the known stirring culture.

Example 5

300mg (dry weight) of adventitious roots obtained in the same manner as in Example 1 was transplanted in a culture tank (capasity, 30cm$^3$) of a culture apparatus consist ing of an aeration tank and the culture tank shown in Fig. 2, followed by culturing for 3 weeks according to a culture method by which a Nitsch-Nitsch liquid medium (dissolved oxygen concentration, 40ppm) containing 3% sucrose and 10$^{-5}$M indole-3-butyric acid supplied with pure oxygen in the aeration tank was supplied at a flow rate of 20mℓ/min. In the culture tank of the present invention shown in Fig. 2, the liquid medium in the culture tank was in a state of a uniform piston flow moving from the lower part to the upper part as indicated with arrows, and thus the adventitious roots in the cultrue tank were in a stable state of giving substantially no vibration. The obtained adventitious roots were subjected to the extraction and the analysis of alkaloid according to the same method as in Example 1. The production of alkaloid in this case was given in Table 5.

Table 5

|  | Dissolved Oxygen Concentration (ppm) | Growth Yield (g*/ℓ) | Yield of Scopolamine (mg/ℓ) | Scopolamine Content (wt %) |
|---|---|---|---|---|
| Example 5 | 40 | 118 | 1350 | 1.2 |

* On dry weight basis.

Example 6

The culture was carried out for 3 weeks in the same manner as in Example 5, except that 150mg (dry weight) of adventitious roots obtained in the same manner as in Example 1. The results were given in Table 6.

Comparative Example 6

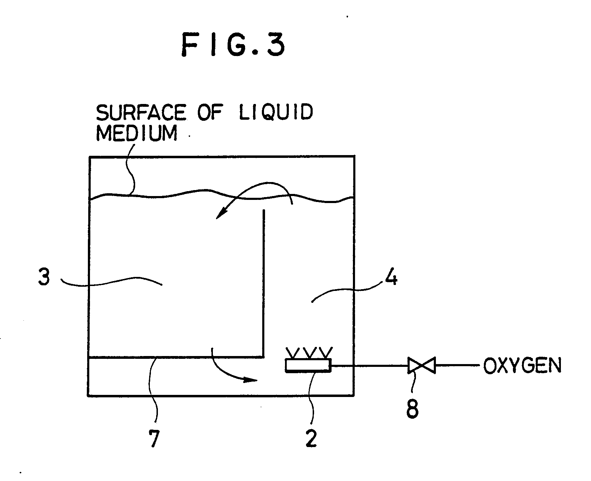

Adventitious roots were cultured in the same manner as in Example 6, except that a culture apparatus shown in Fig. 3 was used. The results were given in Table 6.

In the apparatus shown in Fig. 3, a liquid medium was run from the upper part of an aeration tank to the upper part of a culture tank as indicated with arrows and circulated into the aeration tank from the bottom of the culture tank, were the liquid medium was hard to flow so uniformly as in the case of Example 6 to likely cause channeling. Thus, the productivity of alkaloid was low.

Table 6

|  | Dissolved Oxygen Concentration (ppm) | Growth Yield (g*/ℓ) | Yield of Scopolamine (mg/ℓ) | Scopolamine Content (wt %) |
|---|---|---|---|---|
| Example 6 | 40 | 57 | 630 | 1.1 |
| Comparative Example 6 | 40 | 37 | 100 | 0.27 |

* On dry weight basis.

Example 7

Adventitious roots obtained in the same manner were cultured for 3 weeks by adjusting the dissolved oxygen concentration of a liquid medium to be supplied to a culture tank to 40ppm and changing the amount of adventitious roots to be transplanted variously. The results were shown in Fig. 4 with a line ●-●.

Comparative Example 7

Adventitious roots were cultured in the same manner as in Example 7, except that the dissolved oxygen concentration of a liquid medium to be supplied to a culture tank was adjusted to 8ppm. The results were shown in Fig. 4 with a line O-O.

Example 8

300mg (dry weight) of adventitious root which was the same as used in Example 1 was transplanted to a culture tank of a culture apparatus (capacity, 30cm³) consisting of an aeration tank and the culture tank as shown in Fig. 2 and cultured according to a method by which a Nitsch-Nitsch medium (dissolved oxygen concentration, 40ppm) supplied with pure oxygen in the aeration tank was supplied at a flow rate of 20mℓ/min. (Step 1).

After 1 week from the beginning of culture, the liquid medium was discarded and a Nitsch-Nitsch medium containing 9mM ammonium ion, 36mM sulfuric acid ion, 0.25mM phosphoric acid ion and 5% sucrose but free of indole-3-butyric acid was added thereto anew. The culture was continued for 2 weeks (Step 2).

From the obtained adventitious roots, scopolamine was extracted to measure its content in the same manner as in Example 1. The results were given in Table 7.

Comparative Example 8

The culture of adventitious roots was carried out in the same manner as in Example 8, except that a liquid medium was not discarded after 1 week from the beginning of the culture and a liquid medium was not added anew. The results were given in Table 7.

Table 7

| | Culture Condition | Growth Yield | Scopolamine Content | Yield of Scopolamine |
|---|---|---|---|---|
| | | (g*/ℓ) | (wt %) | (mg/ℓ) |
| Example 8 | Two steps | 115 | 2.1 | 2410 |
| Comparative Example 8 | One step | 118 | 1.2 | 1350 |

* On dry weight basis.

In case of culturing adventitious roots of a plant pro ducing tropane alkaloids according to a method of Example 8, the whole liquid medium in use is changed with a fresh liquid medium in the middle of culture to exclude the influence of waste products exhausted from the tissues to the liquid medium along with the progress of culture. Whereby, the content of tropane alkaloids in the adventitious roots is increase or the growth of the adventitious roots is accelerated to improve the productivity of alkaloids as consequence. Furthermore, the productivity of tropane alkaloids in a cultured tissue can be improved by leaps and bounds by carrying out the culture stepwise by using a liquid medium inducing a lateral root of an adventitious root in Step 1 of the culture and a liquid medium accelerating the elongation of the lateral root and the production of tropane alkaloids.

Example 9

150mg (dry weight) of adventitious roots of *Gynostemma pentaphyllum* Makino was transplanted to a

culture tank (capacity, 30cm$^3$) of a culture apparatus consisting of an aeration tank and the culture tank shown in Fig. 2 and then cultured for 4 weeks according to a method by which a Nitsch-Nitsch medium containing 3% sucrose and $10^{-5}$M indole-3-butyric acid (IBA) which was supplied with pure oxygen in the aeration tank (dissolved oxygen concentration, 40ppm) was supplied at a flow rate of 20ml/min. The growth yield in this case was given in Table 8.

The metabolite obtained here was ginsenoside.

Comparative Example 9

The culture was carried out in the same manner as in Example 9, except that air was supplied instead of pure oxygen (dissolved oxygen concentration, 8ppm) and the amoount of tissues to be transplanted to a culture tank was changed to 15mg (dry weight). The results were given in Table 8.

Table 8

|  | Amount of Tissues to be Transplanted | Growth Yield |
|---|---|---|
|  | (g*/l) | (g*/l) |
| Example 9 | 5 | 71 |
| Comparative Example 9 | 0.5 | 6 |

* On dry weight basis.

Example 10

Using the same culture apparatus as in Example 8, 300mg (dry weight) of seedlings of *Digitalis purpurea* was transplanted to a culture tank of the above apparatus and cultured for 3 weeks according to a method by which a Nitsch- Nitsch liquid medium containing 3% sucrose and $10^{-6}$M kinetin which was supplied with pure oxygen in the aeration tank was supplied at a rate of 20ml/min. The results were given in Table 9.

The metabolite obtained here was digitoxin.

Comparative Example 10

The culture was carried out in the same manner as in Example 10, except that air is used instead of pure oxygen and the amount of seedlings to be transplanted was changed to 30mg (dry weight). The results were given in Table 9.

Table 9

|  | Gas | Amount of Seedlings to be Transplanted (g*/l) | Growth Yield (g*/l) | Digitoxin Content (ppm) |
|---|---|---|---|---|
| Example 10 | Pure Oxygen | 10 | 40 | 40 |
| Comparative Example 10 | Air | 1 | 6 | 6 |

* On dry weight basis.

13

**Claims**

1. A method for culturing a tissue of a plant, characterized by culturing a tissue or a cell of a plant in a culture tank while supplying a liquid medium having a dissolved oxygen concetration of 10ppm or more in a flow state of giving substantially no vibration to the tissue or the cell.

2. A method according to Claim 1, wherein the flow state of giving substantially no vibration to the tissue or the cell is a uniform piston flow.

3. A method according to Claim 1 or 2, wherein the liquid medium is supplied by a forced supply means.

4. A method according to Claim 1, 2 or 3, wherein the tissue or the cell of a plant are proliferated to such a high density as 400g on fresh weight basis/ℓ or more or 20g on dry weight basis/ℓ or more.

5. A method according to Claim 1, 2, 3 or 4, wherein the tissue or the cell of a plant is that of an adventitious root.

6. A method for producing a metabolite, characterized by culturing a tissue or a culture of a plant according to any one of the culture method defined in Claims 1 to 5 and subsequently separating a metabolite from the obtained culture.

7. An apparatus for culturing a tissue of a plant, characterized in that a means for supplying oxygen or a gas containing oxygen into an aeration tank for obtaining an oxygen-dissolved liquid medium and a means for forcedly supplying the oxygen-dissolved liquid medium into a culture tank for a tissue or a cell of a plant from the aeration tank are provided.

8. An apparatus according to Claim 7, wherein a means for preventing the outflow of a tissue or a cell of a plant to be cultured on the outlet side of the oxygen-dissolved liquid medium in the culture tank.

9. An apparatus according to Claim 8, wherein the means for preventing the outflow of the cultured tissue is a filter.

10. An apparatus according to Claim 7, 8 or 9, wherein the means for forcedly supplying the oxygen-dissolved liquid medium to the culture tank from the outside to the inside is a means for supplying the oxygen-dissolved liquid medium in a flow state of giving substantially no vibration to the tissue or the cell of a plant to be cultured.

11. An apparatus according to Claim 10, wherein the means for supplying the oxygen-dissolved liquid medium in a flow state of giving substantially no vibration to the tissue or the cell of a plant to be cultured is a means for supplying the oxygen-dissolved liquid medium to the tissue or the cell of a plant to be cultured by a uniform piston flow.

# FIG.1

# FIG.2

# FIG.3

SURFACE OF LIQUID
MEDIUM

3

4

7

2

OXYGEN

8

# FIG.4